# EUROPÄISCHE PATENTANMELDUNG

(11) **EP 1 936 523 A1**
(43) Veröffentlichungstag der Anmeldung: **25.06.2008**
(21) Anmeldenummer: 06026807.5
(22) Anmeldetag: 22.12.2006
(51) Int. Cl.: G06F 19/00, G06Q 10/00

(54) **System zur Optimierung eines Betreuungs- und Überwachungsnetzwerk**

(71) Anmelder: InterComponentWare AG, 69190 Walldorf (DE)
(72) Erfinder: Heinze, Peter, 69190 Walldorf (DE); Knoop, Hayo, 69190 Walldorf (DE)
(74) Vertreter: Müller-Boré & Partner Patentanwälte

(57) **Zusammenfassung**

Es wird ein System und Verfahren zur computer-implementierten Bestimmung und/oder Steuerung der Auslastung und/oder Dimensionierung eines Betreuungsnetzwerks (10) bereitgestellt. Dabei umfasst das System eine Vielzahl von Zustandsautomaten (36), von denen jeder ausgelegt ist, in Abhängigkeit eines ihm zugeordneten aktiven Parametersatzes (Pₖ) eine Vielzahl von Betreuungsanfragen (38) von jeweils einer durch den aktiven Parametersatz bestimmten Betreuungsart und mit einer durch den aktiven Parametersatz bestimmten Häufigkeit auszugeben; zumindest eine Priorisierungseinrichtung (40) zum Erfassen der Betreuungsanfragen (38) von der Vielzahl der Zustandsautomaten (36), wobei die Priorisierungseinrichtung (40) ausgelegt ist, für jede erfasste Betreuungsanfrage zumindest eine Betreuungsaufgabe mit einem von der jeweiligen Betreuungsart abhängigen Betreuungsvolumen zu generieren und nach einer von der jeweiligen Betreuungsart abhängigen Priorität in zumindest eine Aufgabenwarteschlage (42) einzuordnen; und eine Vielzahl von Betreuungseinheiten (48), welche jeweils eine Betreuungskapazität zur Verarbeitung eines bestimmten Betreuungsvolumens pro Zeiteinheit aufweisen; und eine Zuordnungseinheit zur Zuordnung der Betreuungsaufgaben in der in der Aufgabenwarteschlage (42) angegebenen Reihenfolge und abhängig von freien Betreuungskapazitäten der Betreuungseinheiten (46) und dem Betreuungsvolumen der jeweiligen Betreuungsaufgabe (44) zu den Betreuungseinheiten (46), wobei das System ausgelegt ist, eine Belegung der Betreuungskapazitäten und/oder der Aufgabenwarteschlage auszugeben und/oder zu steuern.

## Beschreibung

Die vorliegende Erfindung betrifft ein System und ein Verfahren zur Vereinfachung der Dimensionierung eines Betreuungs- und/oder Überwachungsnetzwerks, insbesondere eines telemedizinischen Betreuungsnetzwerks.

Mit der Zunahme von bedienerfreundlichen drahtlosen Kommunikationstechnologien ergeben sich neue Möglichkeiten für die Fernüberwachung und -betreuung beispielsweise im telemedizinischen Bereich zur Betreuung von Patienten. Innerhalb telemedizinischer Betreuungs- und Überwachungsszenarien stehen sich verschiedene lose gekoppelte und dynamische Systeme gegenüber. Das Betreuungspersonal leitet die Patienten an, bestimmte Messverfahren wie z.B. Blutdruckmessungen mit einer gegebenen Wiederholfrequenz (z.B. "einmal am Tag") anzuwenden. Aus den durchgeführten Messungen ergeben sich Datensätze die die Grundlage von Einschätzung und Verordnung von Therapiemaßnahmen, z.B. Medikationsänderungen, bilden. Es ist zur Zeit kein System bekannt durch welches das dynamische Verhalten der einzelnen Systemkomponenten im Zusammenspiel optimiert werden kann. Diese Optimierung betrifft insbesondere die Anpassung der Betreuung und Überwachungskapazität an den Bedarf, die Datensicherheit bei der Übertragung von Therapie oder Messdaten oder die Priorisierung von Betreuungsanfragen. Deshalb werden die zur Zeit in diesem Kontext verwendeten Netzwerksysteme aufgrund von "best practice" skaliert und eingesetzt.

Es ist Aufgabe der vorliegenden Erfindung ein System und ein Verfahren zur Vereinfachung bei der Ermittlung bzw. Steuerung der Effizienz und/oder Auslastung und zur Verbesserung der Dimensionierung eines Betreuungsnetzwerks bereitzustellen.

Diese Aufgabe wird durch ein System und ein Verfahren mit den in den unabhängigen Ansprüchen angegebenen Merkmalen gelöst. Bevorzugte Ausführungsformen sind Gegenstand der abhängigen Ansprüchen.

Somit stellt die Erfindung ein System zur computer-implementierten Bestimmung und/oder Steuerung der Auslastung und/oder Dimensionierung eines Betreuungsnetzwerks, insbesondere eines telemedizinischen Betreuungsnetzwerks bereit, welches umfasst:
- eine Vielzahl von Zustandsautomaten, von denen jeder ausgelegt ist, in Abhängigkeit eines ihm zugeordneten aktiven Parametersatzes eine Vielzahl von Betreuungsanfragen von jeweils einer durch den aktiven Parametersatz bestimmten Betreuungsart und mit einer durch den aktiven Parametersatz bestimmten Anfragehäufigkeit, insbesondere einer effektiven bzw. mittleren Anzahl pro Zeiteinheit, auszugeben;
- zumindest eine Priorisierungseinrichtung zum Erfassen der Betreuungsanfragen von der Vielzahl der Zustandsautomaten, wobei die Priorisierungseinrichtung ausgelegt ist, für jede erfasste Betreuungsanfrage zumindest eine Betreuungsaufgabe mit einem von der jeweiligen Betreuungsart abhängigen Betreuungsvolumen zu generieren und nach einer von der jeweiligen Betreuungsart abhängigen Priorität in zumindest eine Aufgabenwarteschlage einzuordnen;
- eine Vielzahl von Betreuungseinheiten, welche jeweils eine Betreuungskapazität zur Verarbeitung eines bestimmten Betreuungsvolumens pro Zeiteinheit aufweisen; und- eine Zuordnungseinheit zur Zuordnung der Betreuungsaufgaben in der in der Aufgabenwarteschlage angegebenen Reihenfolge und abhängig von freien Betreuungskapazitäten der Betreuungseinheiten und dem Betreuungsvolumen der jeweiligen Betreuungsaufgabe zu den Betreuungseinheiten,
wobei das System ausgelegt ist, eine Belegung der Betreuungskapazitäten und/oder der Aufgabenwarteschlage auszugeben und/oder zu steuern.

Dabei repräsentiert bzw. entspricht jeder Zustandsautomat insbesondere einen bzw. einem Betreuten, wie beispielsweise einen Patienten, bzw. jeder Zustandsautomat bildet einen Betreuten ab. Über den aktiven Parametersatz lässt sich ein indikationsspezifisches Verhalten des Patienten in einem Betreuungsnetzwerk festlegen. In Abhängigkeit von diesem indikationsspezifischen Verhalten generiert jeder Zustandsautomat eine Vielzahl von Benachrichtigungen, auf Basis welcher in der Priorisierungseinrichtung jeweils eine Betreuungsaufgabe mit einer vorzugsweise indikationsspezifischen Priorität formuliert wird. Außerdem könnte das Betreuungsvolumen der formulierten Betreuungsaufgabe den für einen insbesondere telemedizinischen Betreuer zur Abarbeitung der Betreuungsaufgabe erforderlichen Zeitaufwand repräsentieren bzw. dem Zeitaufwand entsprechen bzw. ihn abbilden. Dabei repräsentiert bzw. entspricht insbesondere jede Betreuungseinheit einen bzw. einem Betreuer, insbesondere einen telemedizinischen Betreuer, bzw. jede Betreuungseinheit bildet insbesondere einen telemedizinischen Betreuer ab.

Über diese Vorrichtung lässt sich somit in besonders vorteilhafter Weise eine realitätsnahe Bestimmung bzw. Steuerung einer Auslastung eines Betreuungsnetzwerks durchführen sowie Betreuungsnetzwerke geeignet konzipieren und/oder dimensionieren. Besonders vorteilhaft ist die einfache Skalierbarkeit des Systems beispielsweise durch eine Variation der Anzahl von Zustandsautomaten und/oder der Anzahl an Betreuungseinheiten. Außerdem lässt sich in besonders einfacher Weise und in vergleichsweise kurzer Zeit eine langfristige Auslastung eines Betreuungsnetzwerks untersuchen und ggf. an reale und/oder geänderte Gegebenheiten anpassen. Damit kann ein Betreuungsnetzwerk frühzeitig und gezielt an veränderte Gegebenheiten angepasst und optimiert werden. Für jede Aufgabenart könnte bzw. könnten eine Bearbeitungsdauer und/oder Bearbeitungsabläufe festgelegt sein. Die Bearbeitungszeiten bzw. -dauern könnten im jeweiligen Parametersatz festgelegt sein und in einem definierten Intervall schwanken. Eine Spezifikation der Bearbeitungsreihenfolge und -dauer kennzeichnet vorzugsweise das Verhalten einer Betreuungspopulation gegenüber einer Patientenpopulation.

Vorzugsweise ist jedem Zustandsautomat zumindest eine Zustandsklasse zugewiesen, in der jeweils eine Vielzahl von Zustandsstufen definiert ist, wobei jeder Zustandsstufe ein Parametersatz zugeordnet ist und wobei die Zustandsautomaten zumindest teilweise ausgelegt sind, aus der Vielzahl von Zustandsstufen innerhalb der jeweiligen zumindest einen zugewiesenen Zustandsklasse eine aktive Zustandsstufe auszuwählen, deren zugeordneter Parametersatz den aktiven Parametersatz bildet.

Insbesondere für ein telemedizinisches Betreuungsnetzwerk repräsentiert bzw. entspricht vorzugsweise jede Zustandsklasse eine bzw. einer medizinische/n Indikation, wie z.B. eine/r Krankheit oder ein/em Krankheitsbild, bzw. jede Zustandsklasse bildet vorzugsweise eine medizinische Indikation ab. Jede Zustandsstufe repräsentiert bzw. entspricht dabei insbesondere einen bzw. einem Grad der Erkrankung bzw. jede Zustandsstufe bildet einen Grad einer Erkrankung ab. So könnte eine Zustandsklasse beispielsweise Diabetes repräsentieren bzw. abbilden. Die einzelnen Zustandsstufen innerhalb der Zustandsklasse könnten dabei verschiede Grade der Erkrankung repräsentieren bzw. abbilden, welche beispielsweise durch die Zustände "normal", "schwerwiegend", "kritisch" und/oder "nicht messend" ausgedrückt werden könnten.

Das System könnte insbesondere eine Variationskomponente umfassen, welche ausgelegt ist, eine Anzahl der Zustandsautomaten und/oder eine Anzahl der Betreuungseinheiten zu verändern. Diese Veränderung könnte insbesondere in Abhängigkeit von einer ermittelten bzw. ausgegebenen Belegung der Betreuungskapazitäten und/oder der Aufgabenwarteschlage erfolgen. Vorzugsweise wird eine Veränderung der ermittelten bzw. ausgegebenen Belegung der Betreuungskapazitäten und/oder der Aufgabenwarteschlage nach einer Veränderung der Anzahl der Zustandsautomaten und/oder der Anzahl der Betreuungseinheiten von einer Analyseeinheit des Systems ermittelt und insbesondere gespeichert.

Vorzugsweise ist für jede Zustandsklasse eine Vielzahl von Übergangsparametern zwischen den Zustandsstufen innerhalb der jeweiligen Zustandsklasse definiert. Besonders bevorzugt sind die Zustandsautomaten zumindest teilweise ausgelegt, die aktive Zustandsstufe innerhalb derselben Zustandsklasse in Abhängigkeit von den Übergangsparametern zu wechseln. Insbesondere könnte sich ein momentaner Zustand eines Patienten mit einer bestimmten medizinischen Indikation über die Zeit ändern. Somit wird vorzugsweise jeder Patient durch einen Zustandsautomaten abgebildet, der hinsichtlich der Anzahl der Zustände und Transitionen je nach medizinischer Indikation variieren kann. Pro Zustand, insbesondere repräsentiert durch eine Zustandsstufe, wird ein Satz von Parametern definiert, der die Generierung und Verschickung von Nachrichten steuert bzw. bestimmt. Der aktive Zustand bestimmt insbesondere über den zugehörigen Parametersatz zumindest teilweise die vom jeweiligen Zustandsautomaten erzeugten Nachrichten, welche insbesondere durch die Betreuungsanfragen repräsentiert werden.

Ein Zustand, insbesondere repräsentiert bzw. abgebildet durch eine Zustandsstufe innerhalb einer Zustandsklasse, kann über die Zeit entlang der Transitionen, insbesondere zwischen verschiedenen Zustandsstufen innerhalb einer Zustandsklasse, gewechselt werden. Dies wird vorzugsweise vorab durch indikationsspezifische Wahrscheinlichkeiten gesteuert, welche insbesondere durch die Übergangsparameter festgelegt sind. Die Vielzahl der Zustandsautomaten mit jeweils wechselnden aktiven Zustandsstufen repräsentiert bzw. entspricht dabei insbesondere eine/r Vielzahl von Patienten hinsichtlich Ihres Verhaltens innerhalb eines Betreuungsnetzwerks, insbesondere im Hinblick auf ihr Messverhalten von gesundheitsrelevanten Daten und deren Übertragung an das Betreuungsnetzwerk zur Auswertung insbesondere durch telemedizinische Betreuer.

Die von den Patienten verschickten Nachrichten, welche insbesondere durch die von den Zustandsautomaten generierten Betreuungsanfragen repräsentiert bzw. abgebildet werden, bewirken in der Priorisierungseinrichtung das Erzeugen von Betreuungssaufgaben insbesondere für die medizinischen Betreuer. In einer bevorzugten Ausführungsform generiert die Priorisierungseinrichtung für jede Betreuungsanfrage zumindest eine Betreuungsaufgabe. In einer anderen bevorzugten Ausführungsform könnte die Priorisierungseinrichtung auch für mehrere Betreuungsanfragen eines Zustandsautomaten zusammen bzw. basierend auf mehreren Betreuungsanfragen eines Zustandsautomaten eine Betreuungsaufgabe generieren. Nach einem indikationsspezifischen Regelwerk, welches vorzugsweise zumindest teilweise durch die Parametersätze der Zustandsstufen direkt oder indirekt festgelegt bzw. abgebildet oder zumindest beeinflusst ist, können diese Betreuungsaufgaben durch die Priorisierungseinrichtung zur Bearbeitung priorisiert werden. Die erzeugten Aufgaben können für jeden medizinischen Betreuer in Form einer priorisierten Warteschlange umgesetzt werden. Dazu könnte die Priorisierungseinrichtung für jede Betreuungseinheit eine eigene Aufgabenwarteschlage bereitstellen. In einer anderen bevorzugten Ausführungsform wird von der Priorisierungseinrichtung nur eine Aufgabenwarteschlange bereitgestellt.

Vorzugsweise legt der aktive Parametersatz eine spezifische Häufigkeit bzw. Anfragehäufigkeit einer zu generierenden Betreuungsanfrage, insbesondere eine mittlere Anzahl an Betreuungsanfrage pro Zeiteinheit, fest. Besonders bevorzugt sind die Zustandsautomaten ausgelegt, Betreuungsanfragen zu einem zufälligen Zeitpunkt mit der im Parametersatz festgelegten Häufigkeit bzw. Anfragehäufigkeit zu generieren und auszugeben. Insbesondere könnte eine Vielzahl der Parametersätze jeweils eine für die Zustandsstufe spezifische Häufigkeit einer zu generierenden Betreuungsanfrage festlegen.

Vorzugsweise ist die Priorisierungseinrichtung ausgelegt, für jeden Zustandsautomat eine Häufigkeit der von diesem Zustandsautomaten erfassten Betreuungsanfragen zu ermitteln und die Betreuungspriorität für zumindest eine von dem jeweiligen Zustandsautomat erfasste Betreuungsanfrage zumindest teilweise in Abhängigkeit von der ermittelten Häufigkeit festzulegen. Alternativ könnte die Priorisierungseinrichtung die Betreuungspriorität ausschließlich auf Basis eines in der jeweiligen Betreuungsanfrage enthaltenen Prioritätsparameters bestimmen.

Vorzugsweise umfasst das System eine Zustandsdatenbank, welche für die Vielzahl von Zustandsautomaten den zugeordneten aktiven Parametersatz und/oder die Vielzahl von Zustandsklassen umfasst bzw. speichert bzw. bereitstellt. Vorzugsweise umfasst das System außerdem eine Auswerteeinrichtung, welche ausgelegt ist, eine zeitliche Veränderung einer Anzahl von Betreuungsaufgaben in der zumindest einen Aufgabenwarteschlange und/oder eine freie Betreuungskapazität zumindest einer Betreuungseinheit zu erfassen.

Vorzugsweise ist die Auswerteeinrichtung ausgelegt, eine mittlere Verweilzeit der Betreuungsaufgaben in der Aufgabenwarteschlange zu ermitteln. Dies kann für alle Betreuungsaufgaben unabhängig von ihrer Betreuungspriorität erfolgen. Alternativ kann für verschiedene Prioritätsstufen jeweils eine separate effektive bzw. mittlere Verweilzeit bestimmt werden. In einer weiteren bevorzugten Ausführungsform kann auch eine gesamte effektive bzw. mittlere Verweilzeit durch Gewichtung der einzelnen Verweilzeiten abhängig den der jeweiligen Prioritätsstufe bzw. Betreuungspriorität ermittelt werden.

Vorzugsweise ist die Priorisierungseinrichtung ausgelegt, für jede Betreuungsaufgabe insbesondere in Abhängigkeit von der Betreuungsart jeweils zumindest eine Betreuungskompetenz festzulegen bzw. einen Betreuungskompetenzparameter zu umfassen und die Betreuungsaufgaben den Betreuungseinheiten in Abhängigkeit von den Betreuungseinheiten zugewiesenen Betreuungskompetenzen zuzuordnen. Vorzugsweise ist für jede Betreuungskompetenz eine eigene Aufgabenwarteschlage vorgesehen.

Außerdem stellt die Erfindung ein computer-implementiertes Verfahren zur Bestimmung der Auslastung eines Betreuungsnetzwerks, insbesondere eines telemedizinischen Betreuungsnetzwerks, bereit, welches die Schritte umfasst:
- Bereitstellen eines aktiven Parametersatzes, welcher zumindest eine Betreuungsart und zumindest eine Anfragehäufigkeit festlegt;
- Bereitstellen einer Vielzahl von Betreuungseinheiten, welche jeweils eine Betreuungskapazität zur Verarbeitung eines bestimmten Betreuungsvolumens pro Zeiteinheit aufweisen;
- Erzeugen einer Vielzahl von Betreuungsanfragen von der zumindest einen durch den aktiven Parametersatz bestimmten Betreuungsart und mit der zumindest einen durch den aktiven Parametersatz bestimmten Anfragehäufigkeit;
- Generieren zumindest einer Betreuungsaufgabe für jede erzeugte Betreuungsanfrage mit einem von der jeweiligen Betreuungsart abhängigen Betreuungsvolumen und mit einer von der jeweiligen Betreuungsart abhängigen Betreuungspriorität;
- Zuordnen der Betreuungsaufgaben zu den Betreuungseinheiten abhängig von den Betreuungskapazitäten und den Betreuungsvolumina in einer der Betreuungsprioritäten entsprechenden Reihenfolge.

Insbesondere umfasst das Verfahren einen Schritt des Ausgebens und/oder Steuerns einer Belegung der Betreuungskapazitäten und/oder einer Aufgabenwarteschlage. Vorzugsweise umfasst das Bereitstellen eines aktiven Parametersatzes ein Auswählen einer aktiven Zustandsstufe aus einer Vielzahl von Zustandsstufen innerhalb einer Zustandsklasse, wobei jeder Zustandsstufe ein Parametersatz zugeordnet ist. Vorzugsweise umfasst das Auswählen einer aktiven Zustandsstufe ein Wechseln der aktiven Zustandsstufe innerhalb der Zustandsklasse mit einer Wahrscheinlichkeit basierend auf für die Zustandsklasse festgelegen Übergangsparametern.

Vorzugsweise umfasst das Generieren der Vielzahl von Betreuungsanfragen ein Generieren der Betreuungsanfragen zu einem zufälligen Zeitpunkt derart, dass die mittlere Anzahl an generierten Betreuungsanfragen pro Zeiteinheit im Wesentlichen der durch den aktiven Parametersatz bestimmten Anfragehäufigkeit entspricht. Der zufällige Zeitpunkt kann beispielsweise durch einen Zufallsgenerator festgelegt werden.

Vorzugsweise umfasst das Verfahren Schritte des Erstellens zumindest einer Aufgabenwarteschlange für noch nicht verarbeitete Betreuungsaufgaben und des Auswerten einer zeitlichen Veränderung einer Anzahl von Betreuungsaufgaben in der zumindest einen Aufgabenwarteschlange. Vorzugsweise umfasst das Verfahren einen Schritt des Auswertens einer mittleren Verweilzeit der Betreuungsaufgaben in einer Aufgabenwarteschlange vor der Zuweisung der Betreuungsaufgaben zu freien Betreuungskapazitäten der Betreuungseinheiten. Vorzugsweise umfasst das Verfahren einen Schritt des Auswertens einer mittleren freien Betreuungskapazität zumindest einer Betreuungseinheit. Dabei wird vorzugsweise über ein vorgegebenes Zeitintervall gemittelt. Es könnte auch eine mittlere freie Betreuungskapazität für eine Vielzahl von Betreuungseinheiten durch Mitteln über all diese Betreuungseinheiten bestimmt werden.

Vorzugsweise umfasst das Verfahren einen Schritt des Veränderns einer Anzahl an Zustandsautomaten und/oder einer Anzahl an Betreuungseinheiten und einen Schritt des Ermittelns einer Veränderung einer mittleren Verweilzeit der Betreuungsaufgaben in einer Aufgabenwarteschlange bevor die Beteuungsaufgaben freien Betreuungskapazitäten der Betreuungseinheiten und/oder einer freien Betreuungskapazität zumindest einer Betreuungseinheit zugewiesen werden können.

Vorzugsweise umfasst das erfindungsgemäße computer-implementierte Verfahren einen oder mehrere der vom erfindungsgemäßen System zur computer-implementierten Bestimmung und/oder Steuerung der Auslastung und/oder zur Dimensionierung eines Betreuungsnetzwerks oder von einer seiner Komponenten oder einer bevorzugten Ausführungsform davon ausgeführten Verfahrensschritte.

Außerdem stellt die vorliegenden Erfindung ein Computerprogrammprodukt bereit, das, wenn es in einem Computersystem geladen ist, dieses veranlasst, ein Verfahren gemäß der vorliegenden Erfindung oder einer bevorzugten Ausführungsform davon auszuführen.

Die Erfindung wird nachfolgend mit Bezug auf begleitende Zeichnungen bevorzugter Ausführungsformen beispielhaft beschrieben. Dabei zeigt:
- Fig. 1: eine schematische Ansicht eines Betreuungs- und Überwachungsnetzwerks, wie es in einer bevorzugten Ausführungsform der vorliegenden Erfindung Anwendung findet;
- Fig. 2: eine schematische Ansicht eines Systems gemäß einer bevorzugten Ausführungsform der vorliegenden Erfindung; und
- Fig. 3: eine schematische Darstellung eines Systems zur beispielhaften Implementierung der vorliegenden Erfindung in einer bevorzugten Ausführungsform.

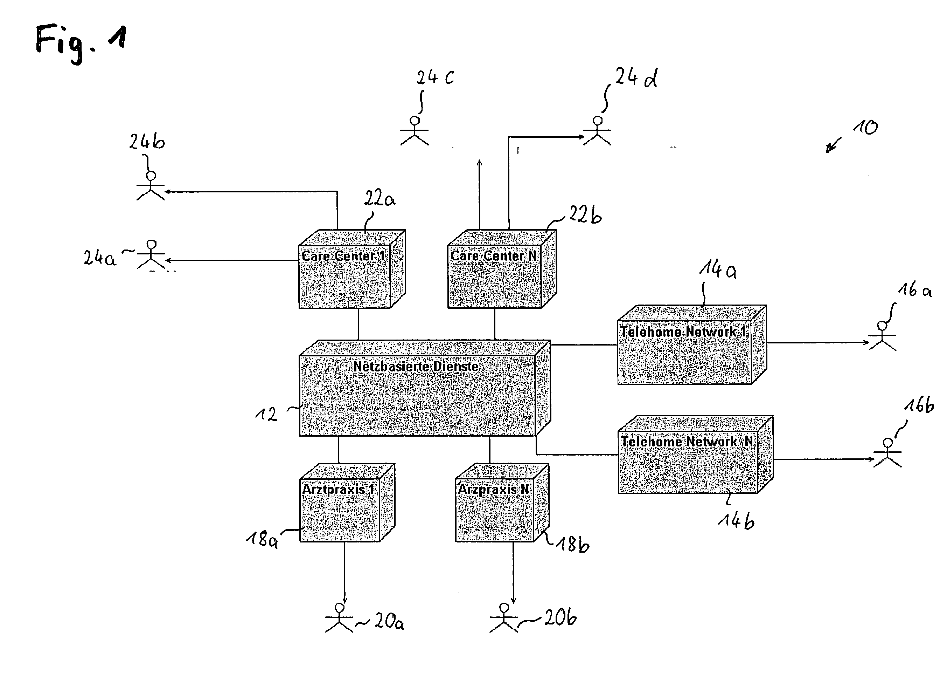

**Fig. 1** zeigt ein Betreuungs- und Überwachungsnetzwerk 10, wie es in einer bevorzugten Ausführungsform der vorliegenden Erfindung Anwendung findet. Das Betreuungs- und Überwachungsnetzwerk 10 umfasst ein Kernnetzwerk 12, über das eine Vielzahl von Peripherienetzwerken und/oder Peripherienetzwerkkomponenten über ein-, bi-, oder multidirektionale Netzwerkverbindungen (insbesondere Internet) gekoppelt sind. Fig. 1 zeigt als ein erstes Peripherienetzwerk ein erstes Patientennetzwerk 14a und ein zweites Patientennetzwerk 14b. In einer bevorzugten Ausführungsform wird das erste und/oder zweite Patientennetzwerke 14a, 14b von einem bzw. über einen Telefonanschluss eines Patienten 16a, 16b gebildet. In einer anderen Ausführungsform könnten die Patientennetzwerke auch von komplexen Telefonsystemen, z.B. einer Telefonanlage einer Pflegeeinrichtungen gebildet werden, über die eine Vielzahl von Patienten auf das Überwachungs- und Betreuungsnetzwerk 10 zugreifen können.

Vorzugsweise sind an das Kernnetzwerk 12 ein oder mehrere Arztnetzwerke 18a, 18b gekoppelt, über die einzelne Ärzte 20a, 20b, z.B. Hausärzte oder Fachärzte, auf das Betreuungs- und Überwachungsnetzwerk 10 zugreifen können. Das Arztnetzwerk 18a, 18b wird vorzugsweise von einem Telefonanschluss und/oder einer Telefonanlage einer Arztpraxis gebildet. Alternativ oder zusätzlich könnte auch ein eigenes, physikalisch von einer Telefonanlage getrenntes bzw. unabhängiges Netzwerk vorgesehen sein.

Schließlich sind in der gezeigten bevorzugten Ausführungsform an das Kernnetzwerk 12 telemedizinische Betreuungsnetzwerke 22a, 22b gekoppelt, über welche vorzugsweise jewells eine Vielzahl von medizinischen Fachbetreuern 24a, 24b, 24c, 24d auf das Betreuungs- und Überwachungsnetzwerk 10 zugreifen können.

Damit können Patienten 16a, 16b über das jeweilige Patientennetzwerk 14a, 14b und das Kernnetzwerk 12 Betreuungsanfragen und/oder medizinische Messdaten an ein geeignetes Betreuungspersonal wie z.B. eine Arztpraxis und/oder ein telemedizinisches Betreuungszentrum übermitteln. Andererseits können über die Arztnetzwerke 18a, 18b bzw. die telemedizinischen Betreuungsnetzwerke 22a, 22b die Haus- oder Fachärzte 20a, 20b bzw. die medizinischen Fachbetreuer 24 Verordnungen von Therapiemaßnahmen an den jeweiligen Patienten 16a, 16b übermitteln.

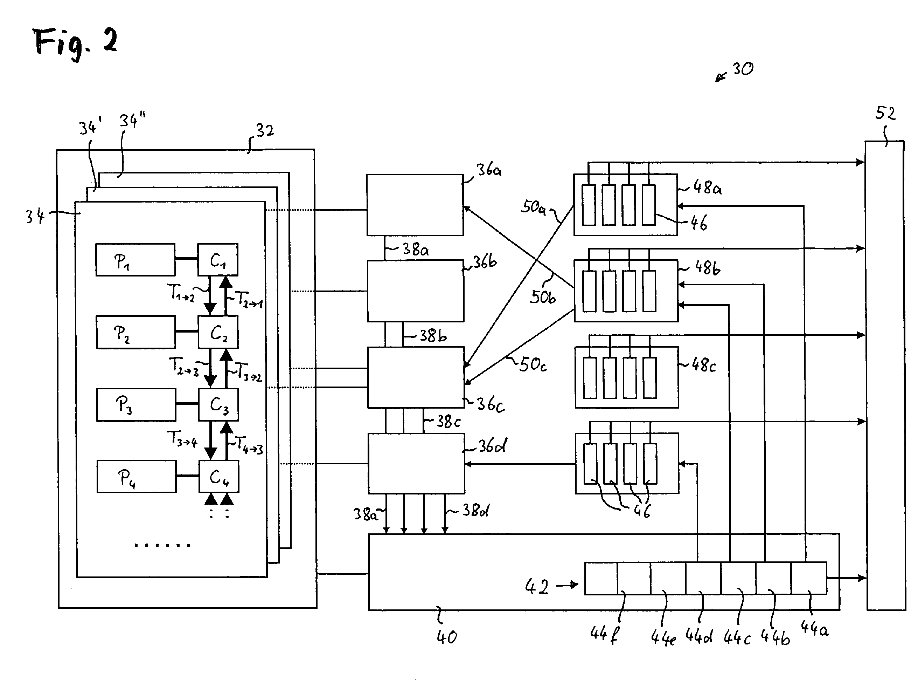

**Fig. 2** zeigt ein System 30 gemäß einer bevorzugten Ausführungsform der vorliegenden Erfindung. In dieser bevorzugten Ausführungsform umfasst das System 30 eine Zustandsdatenbank 32, in der eine Vielzahl von Zustandsklassen 34, 34', 34" definiert ist. Vorzugsweise repräsentiert jede Zustandsklasse 34, 34', 34" eine medizinische Indikation bzw. eine Krankheit bzw. ein Krankheitsbild. So könnte eine erste Zustandsklassen 34 beispielsweise Diabetes repräsentieren, während eine zweite Zustandsklassen 34' Arthritis und eine dritte Zustandsklassen 34" Bluthochdruck charakterisiert. Vorzugsweise ist in jeder Zustandsklasse 34 eine Vielzahl von Zustandsstufen C₁, C₂, C₃, C₄, etc. festgelegt. Vorzugsweise definieren die Zustandsstufen eine Abstufung von Zuständen innerhalb einer Zustandsklasse. Beispielsweise bei einer medizinischen Indikation welche durch die Zustandsklasse 34 bestimmt ist, könnten die Zustandsstufen C₁, C₂, C₃, C₄ unterschiedliche Grade einer Erkrankung repräsentieren. Beispielsweise könnte die Zustands Stufe C₁ für einen Zustand "normal", die Zustandsstufe C₂ für einen Zustand "schwerwiegend", die Zustandsstufe C₃ für einen Zustand "kritisch" und die Zustandsstufe C₄ für einen Zustand "nicht messend" stehen. Außerdem sind Übergangswahrscheinlichkeiten T_{k->l} für einen Wechsel von einer ersten Zustandsstufe Cₖ zu einer zweiten Zustandsstufe Cₗ festgelegt. Vorzugsweise ist jeder Zustandsstufe C₁, C₂, C₃, C₄ ein Parametersatz P₁, P₂, P₃, P₄ etc. zugeordnet. Im jeweiligen Parametersatz sind vorzugsweise typische Werte für Art, Zeit, Häufigkeit und/oder Inhalt einer Betreuungsanfrage festgelegt.

Das System 30 umfasst außerdem eine Vielzahl von Zustandsautomaten 36a, 36b, 36c, 36d. Jedem Zustandsautomat ist zumindest eine Zustandsklasse 34 aus der Zustandsdatenbank 32 zugeordnet. Vorzugsweise repräsentieren die Zustandsautomaten 36 jeweils einen Patienten, dessen Erkrankung durch die zugeordnete Zustandsklasse 34 festgelegt ist. Beispielsweise ist einem ersten Zustandsautomat 36a eine erste Zustandsklasse 34 zugeordnet, welche als medizinische Indikation Diabetes repräsentiert. Der erste Zustandsautomat 36a ist dabei ausgelegt, einen "aktiven Zustand" als eine der Zustandsstufen C₁ ,C₂ , usw. auszuwählen. Außerdem ist der Zustandsautomat 36a ausgelegt den "aktiven Zustand" in Abhängigkeit von den in der Zustandsklasse 34 angegebenen Übergangswahrscheinlichkeiten über die Zeit hinweg zu ändern, wobei die Übergangswahrscheinlichkeiten vorzugsweise durch Übergangsparameter Tₖ bestimmt werden bzw. festgelegt sind. Der Zustandsautomat 36a erfasst in Abhängigkeit vom momentanen "aktiven Zustand" den zugehörigen Parametersatz P aus der Zustandsdatenbank 32 und generiert in Abhängigkeit vom erfassten Parametersatz zumindest eine Betreuungsanfrage 38, welche an eine Priorisierungseinrichtung 40 übertragen wird.

In einer bevorzugten Ausführungsform ist das System ausgelegt, genau eine medizinische Indikation insbesondere durch eine Zustandsklasse zu beschreiben. Dazu ist vorzugsweise allen Zustandsautomaten im Wesentlichen dieselbe Zustandsklasse zugeordnet. Innerhalb der Zustandsklasse wechseln die einzelnen Zustandsautomaten eine aktive Zustandsstufe basierend auf den in der bzw. für die Zustandsklasse festgelegten Übergangsparameter vorzugsweise unabhängig voneinander. In einer anderen bevorzugten Ausführungsform ist eine Vielzahl von Zustandsklassen vorgesehen und zumindest einigen der Zustandsautomaten sind unterschiedliche Zustandsklassen zugeordnet.

Inhalt der Betreuungsanfrage 38, insbesondere eine Betreuungsart, sowie die Häufigkeit, mit der eine Betreuungsanfrage 38 generiert wird, sind vorzugsweise zumindest teilweise im Parametersatz des jeweiligen aktiven Zustands bzw. der jeweiligen aktiven Zustandsstufe Cₖ festgelegt. Außerdem könnte im Parametersatz ein für die Betreuungsart charakteristisches Betreuungsvolumen mit einer Volumenschwankungsbreite festgelegt sein. Beispielsweise könnte für einen aktiven Zustand "schwerwiegend", welcher beispielsweise durch die zweite Zustandsstufe C₂ der Zustandsklasse 34 beschrieben wird, der zweite Parametersatz P₂ einen typischen Blutzuckerwert und dessen Schwankungensbreite sowie eine typische Häufigkeit für die Bestimmung des Blutzuckerwerts für einen schwerwiegend an Diabetes erkrankten Patienten festlegen. Außerdem könnte als Betreuungsart eine individuelle Auswertung der Blutzuckerwerte und als Betreuungsvolumen eine für diese Betreuungsart spezifische bzw. typische Bearbeitungszeit mit einer zugehörigen Schwankungsbreite festgelegt sein.

Vorzugsweise erfasst der erste Zustandsautomat 36a diesen Parametersatz P₂ und generiert mit der darin festgelegten Häufigkeit die Betreuungsanfrage 38, in der vorzugsweise ein vom Zustandsautomaten 36a bestimmter Blutzuckerwert innerhalb des vom Parametersatz P₂ festgelegten Bereichs an die Priorisierungseinrichtung 40 übermittelt wird.

Einem Zustandsautomat können auch mehrere Zustandsklassen zugeordnet sein. So sind dem in Fig. 2 gezeigten dritten Zustandsautomat 36c zwei Zustandsklassen 34 und 34" zugeordnet. Dieser Zustandsautomat 36c könnte dabei einen Patienten repräsentieren, der an zwei Erkrankungen gleichzeitig leidet, das heißt dessen Gesundheitszustand durch zwei verschiedene medizinische Indikationen beschrieben wird. Dabei kann der Zustandsautomat 36c ausgelegt sein, für jede Zustandsklasse 34, 34" eine Zustandsstufe als "aktiven Zustand" unabhängig von der jeweiligen anderen Zustandsklasse auszuwählen. Alternativ könnte in der Zustandsdatenbank 32 auch eine Kopplung der Zustandsstufe zwischen verschiedenen Zuständsklassen festgelegt sein.

Jeder Zustandsautomat 36 generiert und übermittelt in Abhängigkeit von den von der Zustandsdatenbank 32 erfassten Parametersätzen eine Betreuungsanfrage 38 an die Priorisierungseinrichtung 40. In einer bevorzugten Ausführungsform umfasst jede Betreuungsanfrage 38 zumindest einen Priorisierungsparameter, welcher insbesondere die Dringlichkeit der jeweiligen Betreuungsanfrage zum Ausdruck bringt. Die Priorisierungseinrichtung 40 ist dabei ausgelegt, eine Betreuungswarteschlange 42 zu generieren, in der die Betreuungsanfragen in Abhängigkeit von ihren Priorisierungsparametern aufgelistet werden. Die Priorisierungseinrichtung 40 ist dabei ausgelegt, in Abhängigkeit von den erfassten Betreuungsanfrage 38 eine Aufgabenwarteschlange 42 zu generieren, in der eine Reihe von Betreuungsaufgaben 44a, 44b, 44c, 44d, 44e, 44f festgelegt und nach einer Aufgabepriorität angeordnet sind. Vorzugsweise wird für jede Betreuungsanfrage 38 eine Betreuungsaufgabe 44 generiert. Dabei ergibt sich die Aufgabenpriorität vorzugsweise aus dem Priorisierungparameter der jeweiligen Betreuungsanfrage 38. Vorzugsweise ist jede Betreuungsaufgabe 44 durch ihre Aufgabenart sowie eine Aufgabendauer festgelegt. Die Aufgabenart wird vorzugsweise auf Basis der Art und des Inhalts der Betreuungsanfrage 38 generiert. In einer bevorzugten Ausführungsform könnte die Priorisierungseinrichtung 40 mit der Zustandsdatenbank 32 in Kontakt stehen und einige für die Bearbeitung einer Betreuungsanfrage 38 erforderliche Dauer einer Bearbeitungsaufgabe 44 aus dem jeweiligen Parametersatz der zugehörigen Zustandsklasse 34 erfassen. Dazu könnte der Parametersatz P für einen aktiven Zustand C eine typische Dauer der erforderlichen Betreuungsaufgaben sowie eine Variationsbreite dieser Dauer festlegen.

Das System 30 umfasst außerdem eine Vielzahl von Betreuungseinheiten 46, welche in der bevorzugten Ausführungsform in einer Vielzahl von Kompetenzzentren 48 zusammengefasst sind. Dabei sind die Betreuungseinheiten 46 innerhalb eines Zentrums 48 vorzugsweise für zumindest eine spezielle Aufgabenklasse zuständig, die insbesondere einer Zustandsklasse 34 entsprechen könnte. In einer anderen bevorzugten Ausführungsform könnte zumindest ein Kompetenzzentrum auch für eine Vielzahl von Aufgabenklassen bzw. Zustandsklassen zuständig sein.

Die in der Aufgabenwarteschlange 42 aufgelisteten Betreuungsaufgaben werden vorzugsweise in der Reihenfolge ihrer Aufgabenprioritäten nacheinander den verfügbaren Betreuungseinheiten 46 zugewiesen. Vorzugsweise werden die Betreuungsaufgaben dabei in Abhängigkeit von der jeweiligen Aufgabenklasse einem zuständigen Kompetenzzentrum zugeordnet. Nachdem eine Betreuungsaufgabe einer Betreuungseinheit 46 zugewiesen wurde, ist diese Betreuungseinheit 46 für die in der Betreuungsaufgabe 44 festgelegte Zeit für keine weitere Betreuungsaufgabe mehr verfügbar. Ist zu einem Zeitpunkt für eine anstehende Betreuungsaufgabe keine zuständige Betreuungseinheit verfügbar, verbleibt die Betreuungsaufgabe 44 in der Aufgabenwarteschlange 42, bis eine zuständige Betreuungseinheit verfügbar ist.

Die Betreuungseinheiten 46 im System 30 sind außerdem ausgelegt, in Abhängigkeit von der erfassten Betreuungsaufgabe 44 eine Betreuungsantwort 50 an den jeweiligen Zustandsautomat 36 zu übermitteln, auf dessen Betreuungsanfragedaten die Betreuungsaufgabe basiert. Im Falle eines telemedizinischen Betreuungsnetzwerks repräsentiert die Betreuungsantwort vorzugsweise zumindest teilweise eine Therapieverordnung zur Behandlung der durch die Zustandsklasse 34 charakterisierten medizinischen Indikation des durch den Zustandsautomaten 36 dargestellten Patienten. Vorzugsweise ist der Zustandsautomat 36 ausgelegt, die Betreuungsantwort 50 zu erfassen, und in Reaktion auf die erfasste Betreuungsantwort 50 den "aktiven Zustand" innerhalb der jeweiligen Zustandsklasse 34 zu ändern. Vorzugsweise erfolgt eine solche Änderung des "aktiven Zustands" mit einer für den Zustandsautomaten bestimmten Wirkungswahrscheinlichkeit. Beispielsweise könnte sich der "aktive Zustand" des dritten Zustandsautomaten 36c bei Erhalt der Betreuungsantwort 50a mit einer Wirkungswahrscheinlichkeit von beispielsweise 50% von der Zustandsstufe C₂ (z.B. "schwerwiegend") zur Zustandsstufe C₁ (z.B. "normal") ändern. Dieser Wechsel erfolgt in einer bevorzugten Ausführungsform instantan. In einer anderen Ausführungsform erfolgt der Wechsel mit der Wirkungswahrscheinlichkeit innerhalb einer Wirkungszeit oder nach Ablauf einer Wirkungszeit.

In der gezeigten bevorzugten Ausführungsform umfasst das System 30 außerdem eine Auswerteeinrichtung 52, welche mit der Priorisierungseinrichtung 40, insbesondere mit der Aufgabenwarteschlage 42, und mit den Kompetenzzentren 48, insbesondere mit den Betreuungseinheiten 46 zur Auswertung einer zeitlichen Veränderung einer Anzahl von Betreuungsaufgaben in der zumindest einen Aufgabenwarteschlange 42 und/oder eine freie Betreuungskapazität zumindest einer Betreuungseinheit 46 in Signalverbindung steht. Vorzugsweise ist die Auswerteeinrichtung 52 ausgelegt, eine mittlere Verweilzeit der Betreuungsaufgaben in der Aufgabenwarteschlange 42 zu ermitteln.

Vorzugsweise umfasst die Auswerteeinrichtung 52 eine Variationskomponente, welche ausgelegt ist, eine Anzahl der Zustandsautomaten (36) und/oder eine Anzahl der Betreuungseinheiten (46) zu verändern. Diese Veränderung könnte insbesondere in Abhängigkeit von einer ermittelten bzw. ausgegebenen Belegung der Betreuungskapazitäten und/oder der Aufgabenwarteschlage (42) erfolgen. Vorzugsweise wird eine Veränderung der ermittelten bzw. ausgegebenen Belegung der Betreuungskapazitäten und/oder der Aufgabenwarteschlage (42) nach einer Veränderung der Anzahl der Zustandsautomaten (36) und/oder der Anzahl der Betreuungseinheiten (46) von einer vorzugsweise von der Auswerteeinrichtung 52 umfassten Analyseeinheit ermittelt und insbesondere gespeichert.

Für eine medizinische Indikation werden die Parameter für Erzeugung und Versand der Messwerte definiert. Es wird sowohl die Anzahl der Patienten (repräsentiert durch jeweils einen Zustandsautomaten) und die Anzahl der medizinischen Betreuer, als auch die Bearbeitungsdauer der Aufgabenarten mit möglicher Variation festgelegt. So erzeugt dieser Teil des Systems kontinuierlich Nachrichten mit Messwerten, gemäß dieser Einstellungen.

Im zweiten Teil des Systems werden die erzeugten Nachrichten anhand eines Regelwerkes priorisiert und in eine Warteschlange eingereiht. Die Aufgabe mit der jeweils höchsten Priorität belegt den "nehmenden" Betreuer mit der zuvor definierten Bearbeitungszeit. Das Zusammenspiel der beiden Teile des Softwaresystems wird insbesondere durch eine Simulation dargestellt. Die Interpretation der Simulationsergebnisse lässt mehrere Rückschlüsse zu. Es wird vorzugsweise ermittelt, wie viele Patienten mit einer spezifischen medizinischen Indikation sich mit einer gegebenen Betreuungspopulation telemedizinisch betreuen lassen. Außerdem wird vorzugsweise ermittelt, wie viel fachspezifisches Betreuungspersonal notwendig ist, um eine gegebene Anzahl an Patienten mit einer spezifischen Indikation telemedizinisch zu betreuen. Schließlich wird vorzugsweise ermittelt, wie sich systemübergreifende Prozesse vereinfachen lassen, indem Verhaltensmuster geändert oder IT-Systeme geändert bzw. multipliziert werden.

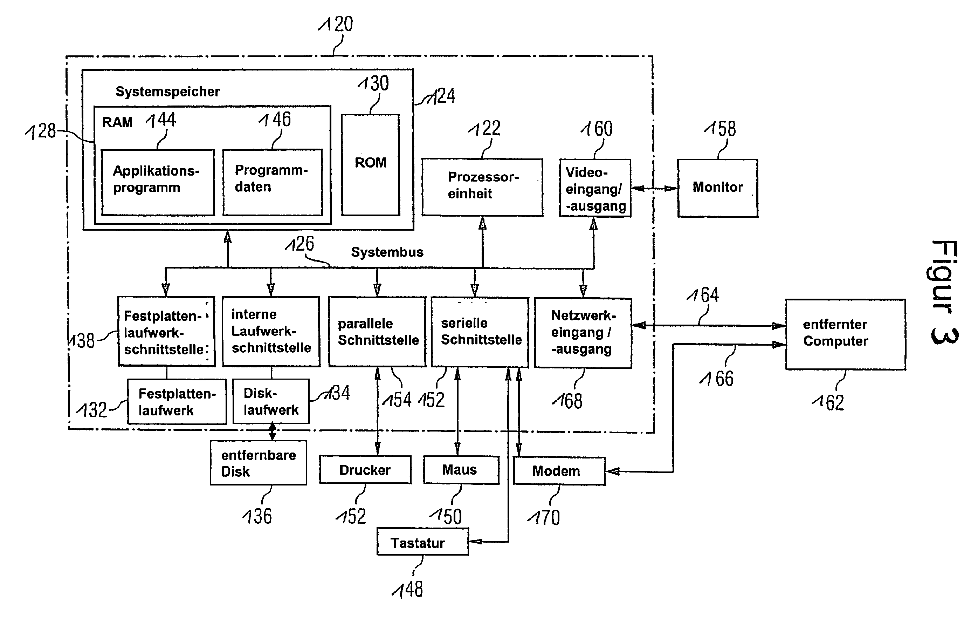

Bezugnehmend auf Fig. 3 wird ein beispielhaftes System zum Implementieren der Erfindung beschrieben. Ein beispielhaftes System umfaßt eine universelle Rechnereinrichtung in der Form einer herkömmlichen Rechnerumgebung 120 z.B. ein "personal computer" (PC) 120, mit einer Prozessoreinheit 122, einem Systemspeicher 124 und einem Systembus 126, welcher eine Vielzahl von Systemkomponenten, unter anderem den Systemspeicher 124 und die Prozessoreinheit 122 verbindet. Die Prozessoreinheit 122 kann arithmetische, logische und/oder Kontrolloperationen durchführen, indem auf den Systemspeicher 124 zugegriffen wird. Der Systemspeicher 124 kann Informationen und/oder Instruktionen zur Verwendung in Kombination mit der Prozessoreinheit 122 speichern. Der Systemspeicher 124 kann flüchtige und nichtflüchtige Speicher, beispielsweise "random access memory" (RAM) 128 und "Nur-Lesespeicher" (ROM) 130 beinhalten. Ein Grund-Eingabe-Ausgabe-System (BIOS), das die grundlegenden Routinen enthält, welche helfen, Informationen zwischen den Elementen innerhalb des PCs 120, beispielsweise während des Hochfahrens, zu transferieren, kann in dem ROM 130 gespeichert sein. Der Systembus 126 kann eine von vielen Busstrukturen sein, unter anderem ein Speicherbus oder ein Speichercontroller, ein peripherer Bus und ein lokaler Bus, welcher eine bestimmte Busarchitektur aus einer Vielzahl von Busarchitekturen verwendet.

Der PC 120 kann weiterhin ein Festplattenlaufwerk 132 zum Lesen oder Schreiben einer Festplatte (nicht gezeigt) aufweisen und ein externes Disklaufwerk 134 zum Lesen oder Schreiben einer entfernbaren Disk 136 bzw. eines entfernbaren Datenträgers. Die entfernbare Disk kann eine magnetische Disk bzw. eine magnetische Diskette für ein magnetisches Disklaufwerk bzw. Diskettenlaufwerk oder eine optische Diskette wie z.B. eine CD-ROM für ein optisches Disklaufwerk sein. Das Festplattenlaufwerk 132 und das externe Disklaufwerk 134 sind jeweils mit dem Systembus 126 über eine Festplattenlaufwerkschnittstelle 138 und eine externe Disklaufwerkschnittstelle 140 verbunden. Die Laufwerke und die zugeordneten computerlesbaren Medien stellen einen nichtflüchtigen Speicher computerlesbarer Instruktionen, Datenstrukturen, Programm-Modulen und anderer Daten für den PC 120 zur Verfügung. Die Datenstrukturen können die relevanten Daten zum Implementieren eines wie oben beschriebenen Verfahrens aufweisen. Obwohl die beispielshaft beschriebene Umgebung eine Festplatte (nicht gezeigt) und eine externe Disk 142 verwendet, ist für den Fachmann offensichtlich, daß andere Typen computerlesbarer Medien, welche computerzugreifbare Daten speichern können, in der beispielhaften Arbeitsumgebung verwendet werden können, wie z.B. magnetische Kassetten, Flash-Memory Karten, digitale Videodisketten, Random-Access-Speicher, Nur-Lesespeicher, usw..

Eine Vielzahl von Programm-Modulen, insbesondere ein Betriebssystem (nicht gezeigt) ein oder mehrere Applikationsprogramme 144, oder Programm-Module (nicht gezeigt) und Programmdaten 146, können auf der Festplatte, der externen Disk 142, dem ROM 130 oder dem RAM 128 gespeichert werden. Die Applikationsprogramme können zumindest einen Teil der Funktionalität, wie in Figur 10 gezeigt, umfassen.

Ein Benutzer kann Kommandos und Information, wie oben beschrieben, in den PC 120 anhand von Eingabevorrichtungen, wie z.B. einer Tastatur bzw. eines Keyboards 148 und einer Computermaus bzw. einem Trackball 150 eingeben. Andere Eingabevorrichtungen (nicht gezeigt) können ein Mikrofon und/andere Sensoren, einen Joystick, ein Spielpolster bzw. -kissen, einen Scanner oder ähnliches umfassen. Diese oder andere Eingabevorrichtungen können mit der Prozessoreinheit 122 anhand einer seriellen Schnittstelle 152 verbunden sein, welche mit dem System 126 gekoppelt ist, oder können anhand anderer Schnittstellen, wie z.B. einer parallelen Schnittstelle 154, eines Spieleports oder eines universellen seriellen Busses (USB) verbunden sein. Weiterhin kann Information mit einem Drucker 156 gedruckt werden. Der Drucker 156 und andere parallele Eingabe/Ausgabevorrichtungen können mit der Prozessoreinheit 122 durch die parallele Schnittstelle 154 verbunden sein. Ein Monitor 158 oder andere Arten von Anzeigevorrichtung(en) ist/sind mit dem Systembus 126 mittels einer Schnittstelle, wie z.B. eines Videoeingang/-ausgangs 160 verbunden. Zusätzlich zu dem Monitor kann die Rechnerumgebung 120 andere periphere Ausgabevorrichtungen (nicht gezeigt) wie z.B. Lautsprecher oder akustische Ausgänge umfassen.

Die Rechnerumgebung 120 kann mit anderen elektronischen Vorrichtungen z.B. einem Computer, einem Schnurtelefon, einem schnurlosen Telefon, einem persönlichen digitalen Assistenten (PDA), einem Fernseher oder ähnlichem kommunizieren. Um zu kommunizieren, kann die Rechnerumgebung 120 in einer vernetzten Umgebung arbeiten, wobei Verbindungen zu einem oder mehreren elektronischen Vorrichtungen verwendet werden. Figur 10 stellt die mit einem "remote computer" bzw. entfernten Computer 162 vernetzte Rechnerumgebung dar. Der entfernte Computer 162 kann eine andere Rechnerumgebung, wie z.B. ein Server, ein Router, ein Netzwerk-PC, eine gleichwertige bzw. "peer" Vorrichtung oder andere gewöhnliche Netzwerkknoten sein und kann viele oder alle der hinsichtlich der Rechnerumgebung 120 oben beschriebenen Elemente umfassen. Die logischen Verbindungen, wie sie in Figur 10 dargestellt sind, umfassen ein "local area network" (LAN) 164 und ein "wide are network" (WAN) 166. Solche Netzwerkumgebungen sind alltäglich in Büros, firmenweiten Computernetzwerken, Intranetzen und dem Internet.

Wenn eine Rechnerumgebung 120 in einer LAN-Netzwerkumgebung verwendet wird, kann die Rechnerumgebung 120 mit dem LAN 164 durch einen Netzwerkeingang/-ausgang 168 verbunden sein. Wenn die Rechnerumgebung 120 in einer WAN-Netzwerkumgebung verwendet wird, kann die Rechnerumgebung 120 ein Modem 170 oder andere Mittel zum Herstellen einer Kommunikation über das WAN 166 umfassen. Das Modem 170, welches intern und extern bezüglich der Rechnerumgebung 120 sein kann, ist mit dem Systembus 126 mittels der seriellen Schnittstelle 152 verbunden. In der Netzwerkumgebung können Programm-Module, welche relativ zu der Rechnerumgebung 120 dargestellt sind, oder Abschnitte davon in einer entfernten Speichereinrichtung gespeichert sein, welche an oder von einem entfernten Computer 162 zugreifbar bzw. systemeigen sind. Weiterhin können andere Daten, welche für das oben beschriebene Verfahren bzw. System relevant sind, auf oder von dem entfernten Computer 162 zugreifbar vorliegen.

### Bezugszeichenliste

- 10: Betreuungs- und Überwachungsnetzwerk
- 12: Kernnetzwerk
- 14a, 14b: Patientennetzwerk
- 16a, 16b: Patient
- 18: Arztnetzwerk
- 20: Arzt
- 22a, 22b: telemedizinische Betreuungsnetzwerke
- 24: medizinische Fachbetreuer
- 30: System
- 32: Zustandsdatenbank
- 34: Zustandsklasse
- 36: Zustandsautomat
- 38: Betreuungsanfrage
- 40: Priorisierungseinrichtung
- 42: Aufgabewarteschlange
- 44: Betreuungsaufgabe
- 46: Betreuungseinheit
- 48: Kompetenzzentrum
- 50: Betreuungsantwort
- 52: Auswerteeinrichtung
- Pₖ: Zustandsparametersatz
- Cₖ: Zustandsstufe
- T_{k->l}: Übergangswahrscheinlichkeit
- 120: Rechnerumgebung
- 122: Prozessoreinheit
- 124: Systemspeicher
- 126: Systembus
- 128: random access memory (RAM)
- 130: Nur-Lesespeicher (ROM)
- 132: Festplattenlaufwerk
- 134: Disklaufwerk
- 136: entfernbare Disk
- 138: Festplattenlaufwerkschnittstelle
- 140: Disklaufwerkschnittstelle
- 142: externe Disk
- 144: Applikationsprogramm
- 146: Programmdaten
- 148: Tastatur
- 150: Computermaus/Trackball
- 152: serielle Schnittstelle
- 154: parallele Schnittstelle
- 156: Drucker
- 158: Monitor
- 160: Videoeingang/ -ausgang
- 162: entfernter Computer
- 164: "local area network" (LAN)
- 166: "wide area network" (WAN)
- 168: Netzwerkeingang/ -ausgang

## Patentansprüche

1. System zur computer-implementierten Bestimmung und/oder Steuerung der Auslastung und/oder Dimensionierung eines Betreuungsnetzwerks (10) umfassend:
- eine Vielzahl von Zustandsautomaten (36), von denen jeder ausgelegt ist, in Abhängigkeit eines ihm zugeordneten aktiven Parametersatzes (Pₖ) eine Vielzahl von Betreuungsanfragen (38) von jeweils zumindest einer durch den aktiven Parametersatz (Pₖ) bestimmten Betreuungsart und mit einer durch den aktiven Parametersatz (Pₖ) bestimmten Anfragehäufigkeit auszugeben;
- zumindest eine Priorisierungseinrichtung (40) zum Erfassen der Betreuungsanfragen (38) von der Vielzahl der Zustandsautomaten (36), wobei die Priorisierungseinrichtung (40) ausgelegt ist, für jede erfasste Betreuungsanfrage (38) zumindest eine Betreuungsaufgabe (44) mit einem von der jeweiligen Betreuungsart abhängigen Betreuungsvolumen zu generieren und nach einer zumindest teilweise von der jeweiligen Betreuungsart abhängigen Betreuungspriorität in zumindest eine Aufgabenwarteschlage (42) einzuordnen;
- eine Vielzahl von Betreuungseinheiten (46), welche jeweils eine Betreuungskapazität zur Verarbeitung eines bestimmten Betreuungsvolumens pro Zeiteinheit aufweisen; und
- eine Zuordnungseinheit zur Zuordnung der Betreuungsaufgaben (44) in der in der Aufgabenwarteschlage (42) angegebenen Reihenfolge und abhängig von freien Betreuungskapazitäten der Betreuungseinheiten (46) und dem Betreuungsvolumen der jeweiligen Betreuungsaufgabe (44) zu den Betreuungseinheiten (46),
wobei das System ausgelegt ist, eine Belegung der Betreuungskapazitäten und/oder der Aufgabenwarteschlage auszugeben und/oder zu steuern.

2. System nach Anspruch 1, wobei jedem Zustandsautomat (36) zumindest eine Zustandsklasse (34) zugewiesen ist, in der jeweils eine Vielzahl von Zustandsstufen (C) definiert ist, wobei jeder Zustandsstufe (Cᵢ) ein Parametersatz (Pᵢ) zugeordnet ist und wobei die Zustandsautomaten (36) zumindest teilweise ausgelegt sind, aus der Vielzahl von Zustandsstufen (C) innerhalb der zugewiesenen Zustandsklasse (34) eine aktive Zustandsstufe (Cₖ) auszuwählen, deren zugeordneter Parametersatz (Pₖ) den aktiven Parametersatz bildet.

3. System nach Anspruch 2, wobei für jede Zustandsklasse eine Vielzahl von Übergangsparametern (T) zwischen den Zustandsstufen (Cᵢ, Cₖ) innerhalb der jeweiligen Zustandsklasse (34) definiert ist, und wobei die Zustandsautomaten (36) zumindest teilweise ausgelegt sind, die aktive Zustandsstufe (Cₖ) innerhalb derselben Zustandsklasse (34) in Abhängigkeit von den Übergangsparametern (T) zu wechseln.

4. System nach einem der vorangegangenen Ansprüche, wobei der aktive Parametersatz (Pₖ) eine spezifische Häufigkeit zumindest einer der zu generierenden Betreuungsanfragen (38) festlegt und wobei die Zustandsautomaten (36) ausgelegt sind, die Betreuungsanfragen (38) zu einem zufälligen Zeitpunkt mit der im Parametersatz (P) festgelegten Häufigkeit zu generieren und auszugeben.

5. System nach einem der vorangegangenen Ansprüche, wobei die Priorisierungseinrichtung (40) ausgelegt ist, für jeden Zustandsautomat (36) eine Häufigkeit der von diesem Zustandsautomat (36) erfassten Betreuungsanfragen (38) zu ermitteln und die Betreuungspriorität für zumindest eine von dem jeweiligen Zustandsautomat erfasste Betreuungsanfrage (38) zumindest teilweise in Abhängigkeit von der ermittelten Häufigkeit festzulegen.

6. System nach einem der vorangegangenen Ansprüche, umfassend eine Zustandsdatenbank (32), welche für die Vielzahl von Zustandsautomaten den zugeordneten aktiven Parametersatz (Pₖ) und/oder die Vielzahl von Zustandsklassen (34) umfasst.

7. System nach einem der vorangegangenen Ansprüche, welches eine Auswerteeinrichtung (52) umfasst, welche ausgelegt ist, eine Anzahl und/oder eine zeitliche Veränderung der Anzahl von Betreuungsaufgaben (44) in der zumindest einen Aufgabenwarteschlange (42) und/oder eine freie Betreuungskapazität zumindest einer Betreuungseinheit (46) und/oder deren zeitliche Veränderung zu erfassen.

8. System nach Anspruch 7, wobei die Auswerteeinrichtung (52) ausgelegt ist, eine mittlere Verweilzeit der Betreuungsaufgaben (44) in der Aufgabenwarteschlange (42) zu ermitteln.

9. System nach einem der vorangegangenen Ansprüche, wobei die Priorisierungseinrichtung (40) ausgelegt ist, für jede Betreuungsaufgabe (44) in Abhängigkeit von der Betreuungsart jeweils zumindest eine Betreuungskompetenz festlegen und wobei das System ausgelegt ist, die Betreuungsaufgaben (44) den Betreuungseinheiten (46) in Abhängigkeit von zumindest einer der jeweiligen Betreuungseinheit (46) zugewiesenen Betreuungskompetenz zuzuordnen.

10. System nach Anspruch 9, wobei für jede Betreuungskompetenz eine eigene Aufgabenwarteschlage (42) vorgesehen ist.

11. Computer-implementiertes Verfahren zur Bestimmung der Auslastung eines Betreuungsnetzwerks umfassend die Schritte:
- Bereitstellen eines aktiven Parametersatzes (Pₖ), welcher zumindest eine Betreuungsart und zumindest eine Anfragehäufigkeit festlegt;
- Bereitstellen einer Vielzahl von Betreuungseinheiten (46), welche jeweils eine Betreuungskapazität zur Verarbeitung eines bestimmten Betreuungsvolumens pro Zeiteinheit aufweisen;
- Erzeugen einer Vielzahl von Betreuungsanfragen (38) von der zumindest einen durch den aktiven Parametersatz (Pₖ) bestimmten Betreuungsart und mit der zumindest einen durch den aktiven Parametersatz (Pₖ) bestimmten Anfragehäufigkeit;
- Generieren zumindest einer Betreuungsaufgabe (44) für jede erzeugte Betreuungsanfrage (38) mit einem von der jeweiligen Betreuungsart abhängigen Betreuungsvolumen und mit einer zumindest teilweise von der jeweiligen Betreuungsart abhängigen Betreuungspriorität;
- Zuordnen der Betreuungsaufgaben (44) zu den Betreuungseinheiten (46) abhängig von den Betreuungskapazitäten und dem jeweiligen Betreuungsvolumen in einer der Betreuungsprioritäten entsprechenden Reihenfolge; und
- Ausgeben einer Belegung der Betreuungskapazitäten und/oder einer Belegung einer Aufgabenwarteschlage (42) durch Betreuungsaufgaben vor deren Zuweisung zu freien Betreuungskapazitäten der Betreuungseinheiten (46).

12. Verfahren nach Anspruch 11, wobei das Bereitstellen eines aktiven Parametersatzes (Pₖ) ein Auswählen einer aktiven Zustandsstufe (Cₖ) aus einer Vielzahl von Zustandsstufen (C) innerhalb einer Zustandsklasse (34) umfasst, wobei jeder Zustandsstufe (Cᵢ) ein Parametersatz (Pᵢ) zugeordnet ist.

13. Verfahren nach Anspruch 12, wobei das Auswählen einer aktiven Zustandsstufe (Cₖ) ein Wechseln der aktiven Zustandsstufe innerhalb der Zustandsklasse (34) mit einer Wahrscheinlichkeit basierend auf für die Zustandsklasse (34) festgelegen Übergangsparametern (T) umfasst.

14. Verfahren nach einem der Ansprüche 11 bis 13, wobei das Generieren der Vielzahl von Betreuungsanfragen (38) ein Generieren der Betreuungsanfragen zu einem zufälligen Zeitpunkt derart umfasst, dass die mittlere Anzahl an generierten Betreuungsanfragen (38) pro Zeiteinheit im Wesentlichen der durch den aktiven Parametersatz (Pₖ) bestimmten Anfragehäufigkeit entspricht.

15. Verfahren nach einem der Ansprüch 11 bis 14, umfassend Schritte des Erstellens zumindest einer Aufgabenwarteschlange (42) für noch nicht verarbeitete Betreuungsaufgaben (44) und des Auswerten einer zeitlichen Veränderung einer Anzahl von Betreuungsaufgaben (44) in der zumindest einen Aufgabenwarteschlange.

16. Verfahren nach einem der Ansprüche 11 bis 15, umfassend einen Schritt des Auswertens einer mittleren Verweilzeit der Betreuungsaufgaben (44) in der Aufgabenwarteschlange (42) vor der Zuweisung der Betreuungsaufgaben (44) zu freien Betreuungskapazitäten der Betreuungseinheiten (46).

17. Verfahren nach einem der Ansprüch 11 bis 16, umfassend einen Schritt des Auswertens einer mittleren freien Betreuungskapazität zumindest einer Betreuungseinheit (46).

18. Verfahren nach einem der Ansprüche 11 bis 17, umfassend einen Schritt des Veränderns einer Anzahl an Zustandsautomaten (36) und/oder einer Anzahl an Betreuungseinheiten (46) und einen Schritt des Ermittelns einer Veränderung einer mittleren Verweilzeit der Betreuungsaufgaben (44) in der Aufgabenwarteschlange (42) vor einer Zuweisung der Beteuungsaufgaben (44) zu freien Betreuungskapazitäten der Betreuungseinheiten (46) und/oder einer freien Betreuungskapazität zumindest einer Betreuungseinheit.

19. Computerprogrammprodukt, das, wenn es in einem Computersystem geladen ist, dieses veranlasst, ein Verfahren gemäß einem der Ansprüche 11 bis 18 auszuführen.
